# EUROPEAN PATENT SPECIFICATION

(11) **EP 2 026 821 B1**
(45) Date of publication and mention of the grant of the patent: **26.08.2009**
(21) Application number: 06755148.1
(22) Date of filing: 11.05.2006
(51) Int. Cl.: A61K 31/728, A61P 19/02

(54) **HYALURONIC ACID BINARY MIXTURES AND THERAPEUTIC USE THEREOF**
BINÄRE HYALURONSÄURE-GEMISCHE UND IHRE THERAPEUTISCHE VERWENDUNG
MÉLANGES BINAIRES D'ACIDE HYALURONIQUE ET UTILISATION THÉRAPEUTIQUE DE CEUX-CI

(43) Date of publication of application: 25.02.2009
(73) Proprietor: Gobbo, Sandra, 1206, Ginevra (CH); Petrella, Robert John, London, Ontario N5X 3W7 (CA)
(72) Inventor: Gobbo, Sandra, 1206, Ginevra (CH); Petrella, Robert John, London, Ontario N5X 3W7 (CA)
(74) Representative: Gervasi, Gemma
(86) International application number: PCT/EP2006/062236
(87) International publication number: WO 2007/131546

(56) References cited:
- WO-A-02/09728
- GOMIS, A. ET AL.: "Effects of Different Molecular Weight Elastoviscous Hyaluronan Solutions om Articular Nocipeptive Afferents" ARTHRITIS AND RHEUMATISM, vol. 50, no. 1, January 2004 (2004-01), pages 314-326, XP002419558 USA
- WEISS C ET AL: "BASIC PRINCIPLES UNDERLYING THE DEVELOPMENT OF VISCOSUPPLEMENTATION FOR THE TREATMENT OF OSTEOARTHRITIS" JOURNAL OF CLINICAL RHEUMATOLOGY, WILLIAMS & WILKINS, BALTIMORE, MD, US, vol. 5, no. 6 SUPPL, 1999, pages S2-S11, XP009078610 ISSN: 1076-1608
- WOBIG, M. ET AL.: "The Role of Elastoviscosity in the Efficacy of Viscosupplementation for Osteoarthritis of the Knee: A Comparison of Hylan G-F 20 and a Lower-Molecular Weight Hyaluronan" CLINICAL THERAPEUTICS, vol. 21, no. 9, 1999, pages 1549-1562, XP002419559 USA

## Description

### Field of the invention

The present invention relates to binary mixtures of hyaluronic acid obtainable with hyaluronic acid samples having different weight-average molecular weight and their use for intra-articular application in joint diseases or extra-articular application in soft tissue disorders.

### Background of the invention

The hyaluronic acid is a well known high molecular weight biological polysaccharide, belonging to the class of glycosaminoglycans, present to a great extent in all connective tissues of vertebrates, in joint synovial fluid and cartilage, where it is a major component, and eye endobulbar fluids, and widely employed for treating a variety of pathological conditions. In nature its molecular weight can reach a molecular weight more than 10,000 kDa, but, as known, when extracted and manipulate the hyaluronic acid is always a sample commonly identified by an average molecular weight, being formed by different polymers having molecular weight comprised in a range of molecular weights. In other word a sample of this biopolymer presents a distribution of molecular weights, so that any sample of HA can be also characterized by an index of polydispersity. Hence, even if not specified, for hyaluronic acid it has to be intended a sample of hyaluronic acid having a determined weight-average molecular weight (or number-average molecular weight) or distribution of molecular weights, when the wording hyaluronic acid or hyaluronan or hyaluronate, usually in form of sodium salt, (hereinafter also indicated as HA) has the meaning of exogenous hyaluronic acid in form of salt.

As for its biomedical application, a lot of samples of this biopolymer extracted from animal sources or bacterial broth are in use for long in pathological conditions such as wound healing, arthrosic/arthritic diseases and ocular surgery, these uses being essentially based on its biological role and on the non-newtonian physical properties (e.g. viscosity and/or its structural viscoelasticity) of its aqueous solutions. Both viscosity and/or viscoelastic properties are mainly dependent on molecular weight range of HA samples, polymer concentration, but other technical features, such as degree of cross-linkage, can contribute to increase the rheological performance as well as the claimed molecular weight.

In fact, the rheological properties of a HA are influenced or resulting from the combination of the different technical features of the sample considered. Notwithstanding, it is common knowledge and practice to refer to a high molecular weight HA samples when a high viscosity and/or viscoelastic properties are requested, in particular, for instance, in knee joint inflammatory conditions, where hyaluronic acid is employed for supplementation purpose of the synovial fluid and for its viscosity/viscoelastic properties.

In fact, the primary role of the endogenous HA in synovial fluid is to provide the adequate mechanical properties of the joint through its viscosity and/or viscoelasticity, then preserving the structure and function of the cartilage articular matrix, while both are affected in articular diseases. For example, osteoarthritis is the result of mechanical and biological events that destabilize the normal degradations synthesis of articular cartilage (Dieppe P. Osteoarthritis. Acta Orthop Scand Suppl. 1998; 281:2-5) and is characterized by a decrease in the concentration and molecular weight of endogenous HA, which in turn may lead to the hallmark signs of pain and loss of function in weight-bearing joints such as the knee (Balasz E.A., Denlinger S.L. Viscosupplementation: a new concept in the treatment of osteoarthritis. J Rheumatol 1993; 20 (Suppl 39):3-9*).* Hence, for intra-articular viscosupplementation/chondroprotection with a HA sample, a direct relationship has been made between an adequate viscosity and/or viscoelasticity and a suitable weight-average molecular weight, preferably a high weight-average molecular weight, at the purpose to restore the concentration and molecular weight characteristics of the synovial fluid for improving pain control and articular function. In clinical trials intra-articular HA preparations have shown pain relief significantly greater than placebo (Dahlberg L., Lohmander L.S., Ryd L. Intraarticular injections of hyaluronan in patients with cartilage abnormalities and knee pain. Arthritis Rheum 1994; 37:521-528*;* Dougados M., Nguyen M., Listrat V., Amor B. High molecular weight sodium hyaluronate (hyalectin) in osteoarthritis of the knee: a 1-year placebo-controlled trial. Osteoarthritis Cartilage 1993; 1:97-103*;* Petrella R.J., DiSilvestro M.D., Hildebrand C. Effects of hyaluronate sodium on pain and physical functioning in osteoarthritis of the knee. Arch Intern Med 2002; 162:292-298*;* Karlsson J., Sjogren L. S., Lohmander L. S. Comparison of two hyaluronan drugs and placebo in patients with knee osteoarthritis: a controlled, randomized, double-blind, parallel-design multi-center study. Rheumatology (Oxford) 2002; 41:1240-1248) and comparable or superior to intra-articular glucocorticoids (Mamlin L.A, Melfi C.A., Parchman M.L., et al. Management of osteoarthritis of the knee by primary care physicians. Arch. Fam. Med. 1998; 7:563-567). Although pain relief is achieved more slowly with HA preparations than with intra-articular glucocorticoid injections, the effect may last considerably longer *(*Adams M.E., Atkinson M.H., Lussier A.J. et al. The role of viscosupplementation with hylan GF-20 (Synvisc) in the treatment of osteoarthritis of the knee: a Canadian multi-centre trial comparing hylan GF-20 alone, hylan GF-20 with non-steroidal anti-inflammatory drugs (NSAIDs) and NSAIDs alone. Osteoarthrits Cartilage 1995; 3:213-226*).* Similarly, intra-articular HA has shown comparable improvement in pain with oral anti-inflammatory preparations (*Adams M.E. et al. 1995 ref. cit.*). Several HA preparations are currently utilized world-wide by clinicians which differ in a consistent manner in molecular weight of HA (from 500,000 to 6x10⁶), dosing regimens and claims of efficacy. Specifically, it is unclear whether differences in efficacy are found among products (Lo G.H., LaValley M., McAlindon T., Felson D. T. Intra-articular hyaluronic acid in treatment of knee osteoarthritis: a meta-analysis. JAMA 2003; 290:3115-21) while patients receive specific products without any objective criteria for a given choice.

Furthermore, it has been described that differences in endogenous HA composition occur among adults with a shift in the viscoelastic properties in osteoarthritis that is consistent with the degree of severity and character of symptoms (*Balasz and Denlinger, 1993 ref. cit.*)*.* The variation of molecular weight estimated from Balasz' paper is from 5 to 2 millions.

The articular disease treatment with relatively low average molecular weight HA may be favoured by the fact that it is mentioned in literature that exogenous HA molecules are possibly trapped or adsorbed in the collagen fibre network. (Balasz E.A., Bloom G.D., Swann D.A. Federation Proceedings 1966, 25, 1813-1816). In relation to the fine structure and glycan content of the surface layer of the articular cartilage, an adsorption of this biopolymer from visco-supplementation administration on an attracting cartilage surface cannot be excluded due to the presence of collagen molecules even by electrostatic interaction. Therefore, in particular, a diffusion of HA molecules in the interfacial layer and adsorption of the polymers, contained in a HA sample having a low or relatively low average molecular weight in relatively high concentration, on the cartilage surface may be considered and may be favoured compared to high weight-average molecular weight.

On the other hand the presence of hyaluronic acid both in cartilage and in synovial fluid highlights a double biological role of endogenous hyaluronic acid, one related with its biological role in cartilage extracellular matrix structural organisation and one related with its physical properties in synovial fluid, and, even considering only the latter aspect the problem of the choice of the best HA sample for joint disease treatment purposes is not easy as expected in spite of the large body of experimental data existing on this polysaccharide. In fact, the knee in dynamic motion requires elastic composition at optimal molecular weight in balance with viscous needs. For example, high frequency loading through synovial fluid is dissipated through a dynamic change in hyaluronic acid toward more elastic modulus compared to more viscous properties when the load to hyaluronic acid is of low frequency (*Balasz and Delinger, 1993 ref. cit.*). While a given HA product has a limited range of molecular weight typically low, medium or high, no product has been designed to provide a complement of composition that mimics the needs of the active osteoarthritic knee joint with reference to elastic and viscous moduli of the synovial fluid. Moreover, viscosity and viscoelastic are recognized to be both useful in the visco-supplementation therapy of the joint diseases, but not necessarily the same sample of HA has both these characteristics adapted for visco-supplementation together, so that it is unforeseeable whatever weight-average molecular weight may be preferable for joint disease treatment. The same problem occurs also in disorders of the soft tissues, such as ligaments, so that also in these cases a right balance between viscosity and viscoelastic properties is necessary.

Therefore, the issue of what is the most appropriate kind of hyaluronic acid with reference to molecular weight and particularly to its resulting physical properties for these treatments is still an open question and the need for a hyaluronic acid preparation having right viscosity and adequate viscoelastic, independently from its weight-average molecular weight, is still unresolved.

### Summary

The present invention provides binary mixtures of hyaluronic acid samples having said HA samples different characteristics with reference to viscosity and/or viscoelastic and mainly different molecular weight and having these HA mixtures demonstrated a right balance between the viscous modulus and viscoelastic modulus for their use, in particular, treating joint diseases.

Therefore, a first object of the present invention is binary mixtures of two samples of hyaluronic acid in form of salt, wherein the rheological features of said binary mixtures at a concentration of 10mg/ml in aqueous solution and at a temperature of 25° C are:
- a flow viscosity of a value at least of 5 Pa/sec at a shear rate of 1/ sec,
- a viscoelastic by dynamic rheology with G' at least of 7.5 Pa and G" at least of 7 Pa at 1 Hz,
and wherein said two hyaluronic acid samples have:
a) the first sample a weight-average molecular weight lower than 1,000,000 Da;
b) the second sample a weight-average molecular weight higher than 1,500,000 Da.

The binary mixtures of hyaluronic acid samples of the invention are suitable for restoring synovial fluid elasticity and biological lubrication, then a further object of the present invention provides their use for the inflammatory and/or traumatic joint disease treatment or as adjuvant in arthroscopy by intra-articular application and for disorders of soft tissues by extra-articular application.

These and other objects as well as features and advantages of the present invention will be better understood from the detailed description set forth below.

### Brief description of the drawings

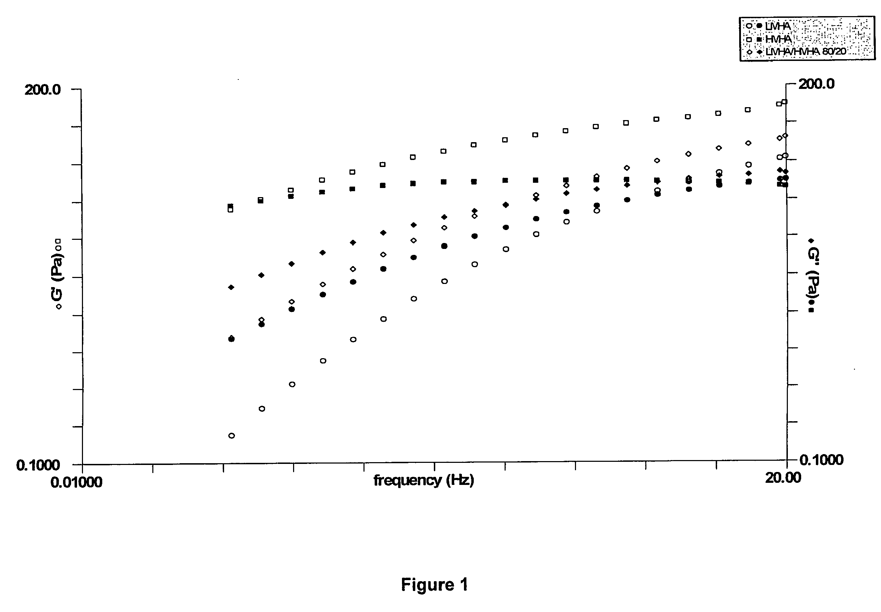

Figure 1 shows the dynamic rheology G'/G" for a low weight-average molecular weight hyaluronic acid (LMHA O, ●), a high weight-average molecular weight hyaluronic acid (HMHA ■, □) and the mixture of the same LMHA:HMHA (◆, ◊) in a w/w ratio of 80:20. It is clear that the transition G'=G" is shift to lower frequency and increases the elastic behaviour of LMHA solution in the presence of HMHA.

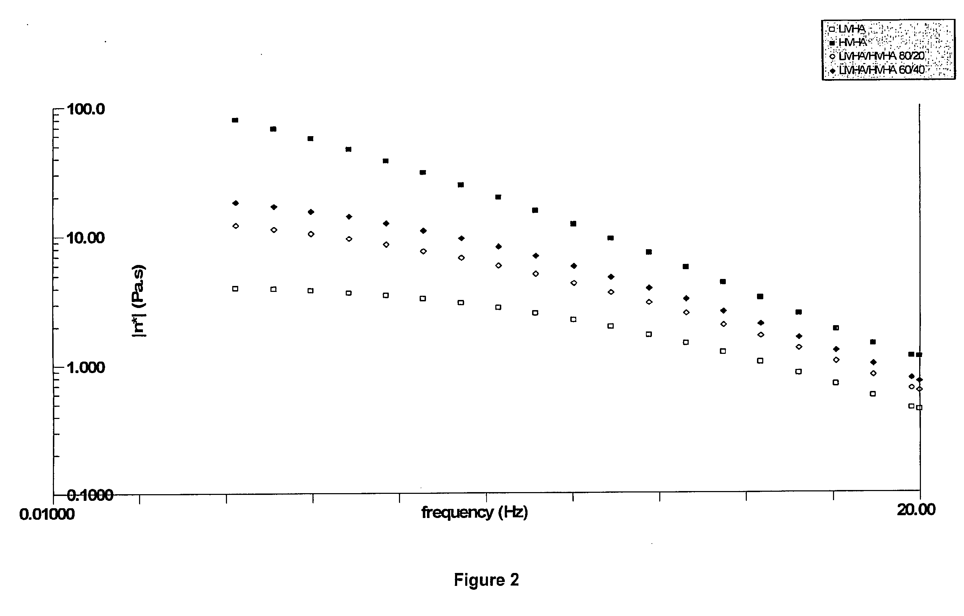

Figure 2 shows the influence of the composition of the mixture on the complex viscosity as a function of the frequency of a low weight-average molecular weight hyaluronic acid (LMHA □), a high weight-average molecular weight hyaluronic acid (HMHA ■), the mixtures of the same LMHA:HMHA in a w/w ratios of 80:20 (◊) and of 60:40 (◆).

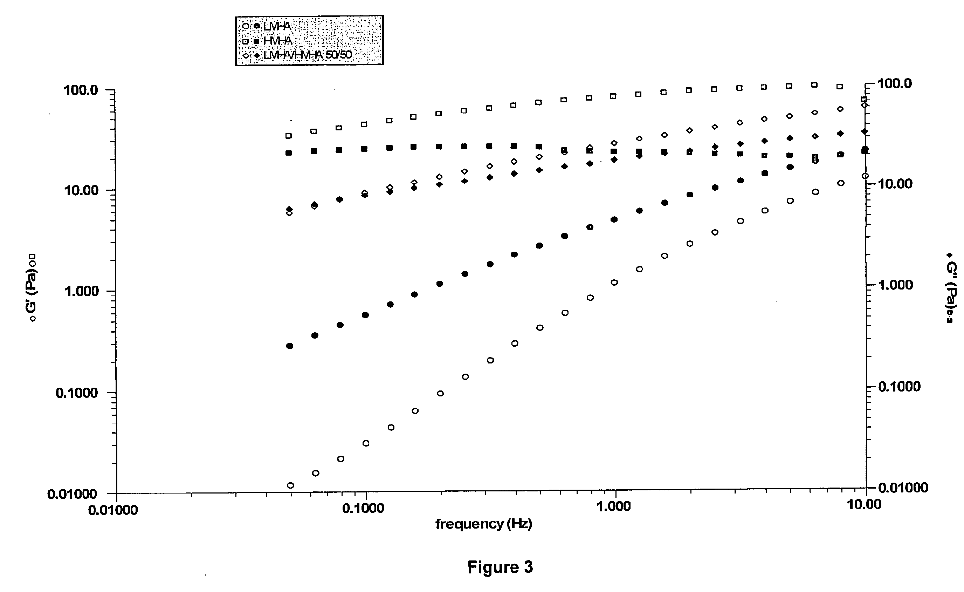

Figure 3 shows the dynamic rheology for a low weight-average molecular weight hyaluronic acid (LMHA ○, ●), a high weight-average molecular weight hyaluronic acid (HMHA □, ■) and the mixture of the same LMHA:HMHA in a w/w ratio of 50:50 (◆, ◊). It is clear a transition G'=G" appears at low frequency and increases the elastic behaviour of LMHA solution upon addition of HMHA.

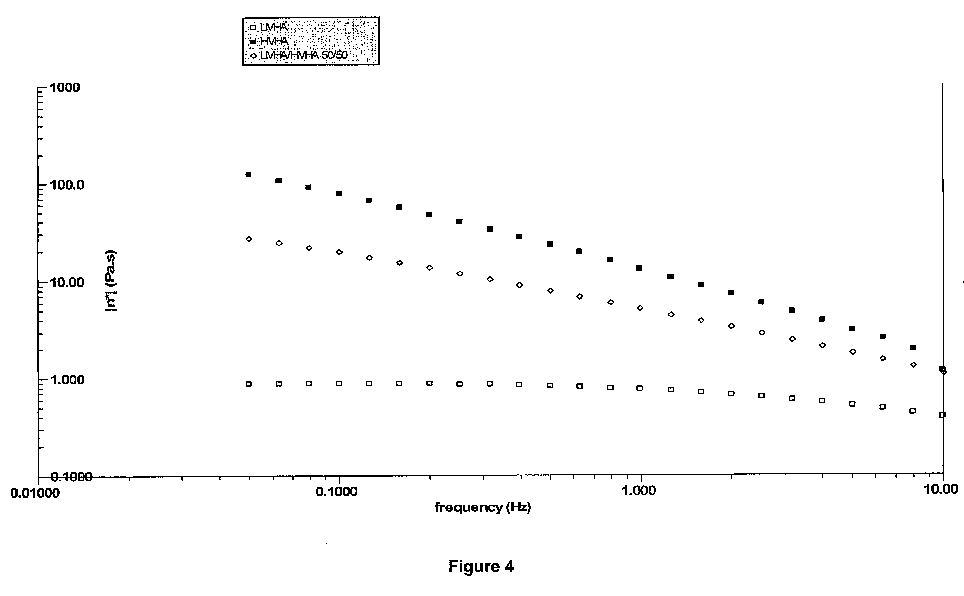

Figure 4 shows the influence of the composition of the mixture on the complex viscosity as a function of the frequency of a low weight-average molecular weight hyaluronic acid (LMHA □), a high weight-average molecular weight hyaluronic acid (HMHA ■) and the mixture of the same LMHA:HMHA in a w/w ratio of 50:50 (◊).

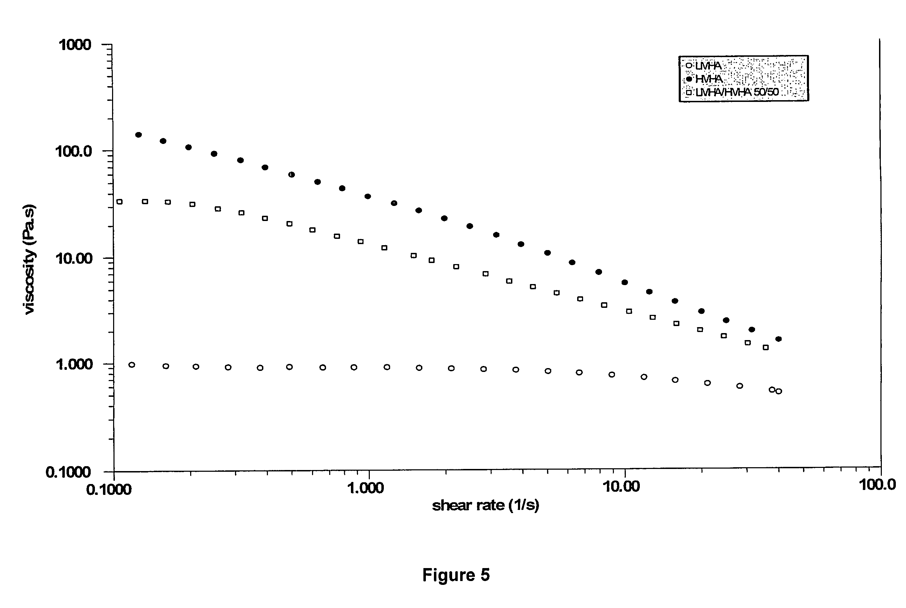

Figure 5 shows the flow viscosity of a low weight-average molecular weight hyaluronic acid (LMHA ○), a high weight-average molecular weight hyaluronic acid (HMHA ●) and the mixture of the same LMHA:HMHA in a w/w ratio of 50:50 (□) as a function of the shear rate.

### Detailed description of the invention

The features and the advantages of the invention will become apparent in the following description and from the preferred embodiments which are given for illustration and no limiting purposes. Further implementation or adaptations as well as embodiments readily apparent to those skilled in the art are to be considered within the scope of the present invention.

The present invention allows to overcome the drawbacks of existing preparations of hyaluronic acid for joint disease treatment through binary mixtures of hyaluronic acid samples wherein one HA sample of said mixtures confers biological lubrification properties and the second one, elasticity of the synovial fluid.

As previously mentioned, samples of HA, having molecular weights from 500,000 to 6x10⁶ Da, are employed for long for their viscosity and/or viscoelasticity in viscosupplementation therapy of joint diseases. However the right balance between the viscous contribution for lubrication of the joint and viscoelasticity for the dynamic motion of the same is not fulfilled in an adequate manner. In fact it is well known that, while samples of HA having low molecular weight exhibit a prevailing viscous behaviour, samples of HA having higher molecular weight exhibit a prevailing visco-elastic behaviour. Other technical features, such as polymer concentration, degree of cross-linkage, can contribute to increase the rheological performance as well as the claimed molecular weight.

The Applicants have now found that mixing together two samples of HA having different weight-average molecular weight and then different viscosity/viscoelastic profile in aqueous solution, the resulting HA binary mixtures have a balance between the viscous modulus and viscoelastic modulus adapted for the viscosupplementation in joint disease treatment or in soft tissue disorders, being the viscosity essential for biological lubrication and the viscoelastic property essential for dynamic motion of the joint. Therefore compositions of pharmaceutical grade of hyaluronic acid binary mixtures of the invention are particularly useful for intra-articular application in joint inflammatory and/or traumatic diseases or extra-articular application in soft tissue disorders, solving the problem above mentioned of viscosity and viscoelasticity combining the right characteristics with reference to viscosity and viscoelastic behaviour in the same product.

To fulfil the purpose of the present invention, the hyaluronic acid mixtures are binary mixtures having at a concentration of 10mg/ml in aqueous solution and at a temperature of 25°C:
- a flow viscosity of a value at least of 5 Pa/sec at a shear rate of 1/sec,
- a viscoelasticity by dynamic rheology with G' at least of 7.5 Pa and G" at least of 7 Pa at 1 Hz,
and preferably
- a flow viscosity of a value in a range comprised from 5 to 25 Pa/sec at a shear rate of 1/sec,
- a viscoelasticity by dynamic rheology with G' in a range comprised from 7.5 to 30 Pa and G" in a range comprised from 7 to 20 Pa at 1 Hz.

The most preferred rheological features of said binary mixtures are at a concentration of 10mg/ml and at a temperature of 25°C:
- a flow viscosity of a value in a range comprised from 10 to 15 Pa/sec at a shear rate of 1/sec,
- a viscoelasticity by dynamic rheology with G' in a range comprised from 10 to 15 Pa and G" in a range comprised from 7 to 10 Pa at 1 Hz.

In one embodiment of the present invention the HA binary mixtures are obtainable with hyaluronic acid samples having different weight-average molecular weights.

In particular, the sample of HA, having a lower weight-average molecular weight, has a weight-average molecular weight lower than 1,000,000 Da (LMHA) and preferably in a range comprised from 300,000 to 1,000,000 Da and most preferably of 500,000 - 900,000 Da weight-average molecular weight. Generally, in these ranges of molecular weight the hyaluronic acid samples have respectively a viscosity around 1 Pa.s at 0.1s⁻¹ and an elastic modulus G'∼1Pa, lower than G" at 1 Hz.

The second sample of HA, having higher weight-average molecular weight, has a weight-average molecular weight not less than 1,500,000 Da (HMHA) and preferably in a range comprised from 1,500,000 up to 6,000,000 Da and preferably between 1,800,000 and 3,500,000 Da weight-average molecular weight. Generally, in these ranges of molecular weight the hyaluronic acid samples have respectively a viscosity larger or equal to 70 Pa.s at 0.31s⁻¹ and a viscoelastic G' modulus larger than 65 Pa.

The HA binary mixtures, having the rheological features in term of flow viscosity and viscoelasticity by dynamic rheology suitable to fulfil the purpose of the invention, can be obtained with the two samples of HA herein above mentioned in adequate w/w ratios between themselves.

It is one of the preferred embodiment of the invention a binary mixture comprising two hyaluronic acid in form of salt samples, wherein the rheological features of said binary mixture are at a concentration of 10mg/ml in aqueous solution and at a temperature of 25°C:
- a flow viscosity of a value in a range comprised from 5 to 25 Pa/sec at a shear rate of 1/sec,
   - a viscoelasticity by dynamic rheology with G' in a range comprised from 7.5 to 30 Pa and G" from 7 to 20 Pa at 1 Hz
and wherein said two hyaluronic acid samples have:
a) the first sample a weight-average molecular weight in a range comprised from 300,000 up to 1,000,000 Da,
b) the second sample a weight-average molecular weight in a range comprised from 1,500,000 up to 6,000,000 Da,
being said binary mixture obtainable by mixing the hyaluronic acid in form of salt sample a) and hyaluronic acid in form of salt sample b) in a w/w ratios in ranges comprised from 80:20 to 20:80.

Other preferably w/w ratios of the hyaluronic acid sample a) and hyaluronic acid sample b) are in ranges comprised from 40:60 to 60:40 and most preferably the w/w ratio is 50:50.

For the purposes of the invention the HA samples used for the preparation of the binary mixtures are preferably, but not exclusively, linear hyaluronic acids, while the salts thereof are salts alkaline metals and preferably Na or K.

For intra-articular application in joint inflammatory and/or traumatic diseases or extra-articular application in soft tissue disorders, the compositions of hyaluronic acid binary mixtures of the invention can be composition of pharmaceutical grade in association with diluents and excipients acceptable for the foreseen use, preferably buffered and solvating medium wherein the hyaluronic acid is in concentration from 10mg/ml to 20 mg/ml. In a preferred embodiment the hyaluronic acid is in concentration of 10mg/ml or 20 mg/ml. Preferably the diluents are buffered solvating media at physiological pH with a adequate osmolarity.

The experimental part given hereinafter consists in a characterization of the feature of the physical properties (i.e rheology) of the selected HA samples commercially available and of the binary mixtures thereof (part A) and in a clinical study on patients with osteoarthritis performed with HA pharmaceutical products also commercially available and employable for regulatory issues (part B).

In order to characterise and evaluate the rheological behaviour of the hyaluronic acid mixtures of the invention in the experiments performed, the response to stress is determined and the elastic contribution is measured in the same time as the viscous contribution as a function of pulsation (rad/s) or frequency (Hz) (ω).

In fact, the intrinsic viscosity can be obtained at zero shear rate and zero polymer concentration and it is well known that an empirical relationship exists between the intrinsic viscosity in a given solvent and a given temperature and the molecular weight (Mark-Houwink relation [η](mL/g)= 0.0336M ^{0.79} for HA in 0.15M NaCl).

Moreover, the viscosity of a solution depends also directly on this intrinsic viscosity and on the polymer concentration at low shear rate. Over a given critical polymer concentration (depending on these two factors), the chains overlap and one gets non Newtonian behaviour in flow experiments. Therefore, the most important technical feature to evaluate for a polymer solution, and in particular for hyaluronic acid solutions, is the viscoelasticity characterizing the polymer solution (obtained in dynamic rheological experiments where the frequency or pulsation imposed for the stress applied on the solutions varies when the linear conditions are respected).

### EXPERIMENTAL PART

### PART A: Rheological behaviour of HA binary mixtures

Materials and methods. For the study different samples of hyaluronic acid have been employed: ARD sample (lot n° 03167) produced by ARD having a MW as declared by the producer around 10⁶ (sample 1); HyluMed sample (Genzyme, lot 690113) having a MW as declared by the producer of 2.59x10⁶ (sample 2); Hylan GF20 or Synvisc (Genzyme) containing 20% crosslinked HA and 80% linear HA having a MW as declared by the producer of 6x 10⁶ (sample 3); Suplasyn (Bioniche) a linear HA from bacterial source having a MW as declared by the producer of (MW 500,000- 1,000,000 Da) (sample 4); mixtures of ARD and HyluMed samples of 80/20 (sample 5); mixtures of 60/40 (sample 6); mixtures of Hylan GF20 or Synvisc and Suplasyn of 50:50 (sample 7).

The viscosity of the HA samples, being not declared by the producers, has been measured in determined conditions of temperature, solvent, shear rate.

On these samples at concentration of 10mg/ml in 0.15M NaCl in aqueous solution at temperatures of 25°C the rheological behaviour has been analysed with a TA Instrument rheometer equipped with a plane-cone geometry; the cone was 4cm diameter and 3.59 degree angle. This equipment allows to perform: (1) flow experiment (determination of the viscosity in Pa.s as a function of the shear rate expressed in s⁻¹) and (2) dynamic experiments, measuring the storage (G') and loss modulus (G") expressed in Pa. as a function of the frequency of deformation (ω in Herz); G' represented the elastic behaviour of the solution under stress (solid like character) and G", the viscous character. For a viscoelastic solution, one gets G">G' at low frequency , then G' and G" cross at ω₀ and G' becomes larger than G" at high frequency. The complex viscosity |η*| (in Pa.s) is also calculated and combines G' and G''. To restore viscoelasticity of the synovial fluid, it is needed to prepare a solution having ω₀ lower than 0.5 Hz (corresponding to walking frequency as given by Balazs (Balazs E.A., Gibbs D.A. "The rheological properties and function of hyaluronic acid. In Chemistry and molecular biology of the intracellular matrix"" edit. Balazs E.A., Academic press, London and NY, 1970 ,1241-1254).

Steric exclusion chromatography (Waters GPCV Alliance 2000) equipped with 3 detectors on line (refractometer, Wyatt MALLS light scattering, viscometer) was used to determine in absence of calibration the weight -average molecular weight and all the molecular weight distribution, being this the only technique valid to determine the molecular weight in our view. The samples were prepared at 0.5 g/L in 0.1M NaNO3 eluent and filtered through a porous membrane (0.2 micrometer pore diameter) before injection of 108 microliters.

Different mixtures can be prepared by mixed controlled amount of each HA samples such as preserve the total polymer concentration fixed at 10 g/L. HMHA imposes the position of ω₀ at relatively low value going to the critical value of 0.5 Hz; LMHA allows to preserve the total amount of HA at injection but also is suppose to better interact with the cartilage to form an interfacial zone rich in HA.

The results obtained are reported in table 1 for ARD and Genzymes samples and binary mixtures thereof and in table 2 for Synvisc and Suplasyn samples and mixtures thereof employed in the clinical trial hereinafter reported.

**Table 1. Influence of mixture composition on properties. Solution 10g/L in 0.15M NaCl, T=25°C. (ARD LMHA/Genzyme HMHA)**

| Solutions | Viscosity | G' Pa. at 1 Hz | G" Pa. at 1 Hz | ω₀ (Hz) | \|η*\| | Mw |
|---|---|---|---|---|---|---|
| | Pa.s at 0.1 s-1 | | | | Pa.s. at 1 Hz | (g/mol) |
| LMHA | - | 7.36 | 11.38 | 3.6 | 2.23 | 1x10⁶ |
| HMHA | - | 67.28 | 29.93 | 0.06 | 12.13 | 2.59x10⁶ |
| Mixture | - | 26.62 | 22.95 | 0.6 | 5.79 | |
| LMHA/HMH | | | | | | |
| A 60/40 | | | | | | |
| Mixture | - | 18.29 | 18.61 | 1 | 4.3 | |
| LMHA/HMH | | | | | | |
| A 80/20 | | | | | | |

The same results are shown in fig. 1-2. In figure 1, it is shown that Genzyme sample is very viscoelastic when ARD sample is less entangled. The frequency ω₀ for the transition in G', G" is located at a much higher value for ARD (3.7 Hz > 0.064 Hz for Genzyme) but at the same value of modulus G'=20 Pa. In figure 2, the complex viscosity as a function of the shear rate is given for the same solution;

it shows the normal decrease with increasing the shear rate for viscoelastic entangled solutions.

**Table 2. Influence of mixture composition on properties. Solution 10g/L in 0.15M NaCl, T=25°C.(Synvisc HMHA /Suplasyn LMHA)**

| Solutions | Viscosity | G' Pa at 1 Hz | G" Pa at 1 Hz | ω₀ | Mw (g/mol) | \|η*\| Pa.s. at |
|---|---|---|---|---|---|---|
| | Pa.s at 0.1 s-1 | | | (Hz) | | 1 Hz |
| LMHA | 0.96 | 1.08 | 4.60 | - | 500,000- | 0.76 |
| | | | | | 1,000,000 | |
| HMHA | 159.9 | 78.63 | 21.89 | - | Given at 6x10⁶ | 13 |
| Mixture | 43.2 | 26.38 | 18.17 | 0.07 | | 5.12 |
| LMHA/HMHA | | | | | | |
| 50/50 | | | | | | |

The same results are shown in fig. 3-5. In figure 3, the dynamic moduli are given for the initial solutions and the 50/50 % mixture at 10 g/L. Synvisc is elastic in all the domain of frequencies covered; Suplasyn is mainly viscous in relation with the relatively low molecular weight with no cross point of the moduli. The 50/50% mixture has a very interesting behaviour, being mainly elastic in a large range of frequencies with a cross point at ω₀ =0.075 Hz much lower than the walking frequency 0.5Hz. In figures 4 and 5 , the complex viscosity |η*| and the viscosity η are given as a function of the shear rate.Suplasyn shows a Newtonian plateau due to its relatively low molecular weight.

### PART B: Intra-articular hyaluronic acid (binary) mixtures in patients with osteoarthritis of the knee

At the purpose to establish the advantage of the HA mixtures of the invention respect to the several products ranging from lower to higher molecular weight used at present with no clear difference in efficacy, a clinical study on patients with osteoarthritis of the knee has been conducted.

Method. Twenty-three patients (age 68±8y; mean duration of symptoms 7.4±4.1y) referred for management of osteoarthritis of the knee who were not naive to intra-articular hyaluronic acid therapy and met the entry criteria including resting visual analogue scale (VAS) pain of >45 mm, radiographic confirmation of unilateral knee grade 1-3 osteoarthritis and willingness to receive intra-articular hyaluronic acid therapy as a single or combined range of molecular weight were enrolled in a randomized, prospective, single-blind naturalistic cohort followed for 12 weeks study. Patients were randomly selected at baseline to receive a three intra-articular injection series with one of Suplasyn (10mg/ml; 2ml/injection, MW 500,000-1,000,000 Da in the following identified as LMW), Synvisc (8mg ± 2/1mL; 2ml/ injection, MW 6 million Da in the following identified as HMW), or combined Suplasyn-Synvisc (15mg/1.5mL +12mg ±3/1.5ml; 3 ml/injection in the following identified as L-HMW) over 3 weeks. Patients were free to use concomitant medications or physiotherapy at their own discretion.

Patients completed baseline assessment of rest and walking VAS pain (primary efficacy variable), collection of a 5-point categorical global satisfaction score and record of adverse events and concomitant therapeutic modality use at each study visit and one week after the last injection. Patients were instructed to return for repeat measurement including adverse events at 12 weeks.

Results. The patients were randomized to one of the three groups [LMW=9; HMW=6; L-HMW=8]. No difference in baseline demographics, grade of osteoarthritis symptoms, prior use of either lower or higher MW product use or baseline efficacy outcomes were observed between groups. Two subjects (LMW; L-HMW) dropped out for non-study related reasons. At 4 and 12 weeks, there were no differences between any of the groups in adverse events (<1.3% overall) limited to pain and local swelling. At 4 and 12 weeks respectively, the change in walking VAS pain was significantly improved from baseline in all three groups:

LMW (81.3%, p<0.001; 63.6%, p<0.001); HMW (78.1%, p<0.001; 59.1%, p<0.001) and L-HMW(50/50 mixture) (89.3%, p<0.001; 79.6%, p<0.001). Patients in L-HMW group had significantly greater improvement at both 4 and 12 weeks (p<0.007) compared to the other groups who did not differ from each other. Similarly, rest VAS pain was significantly decreased in all 3 groups from baseline at 4 and 12 weeks, however, there was no significant difference among groups. Use of concomitant therapeutic modalities at 4 weeks was not different among groups however, significantly more LMW patients used concomitant therapeutic modalities at 12 weeks compared to the other 2 groups (p<0.05). Global satisfaction was significantly higher for the L-HMW compared to the other groups at 12 weeks (p<0.005).

Conclusions. Intra-articular hyaluronic acid injections using any of low, high or combined MW were highly effective in improving resting and more so, walking pain in patients with osteoarthritis of the knee. Greater improvement in both rest and activity outcomes in patients who received the combined L-HMW group, with concomitant greater patient satisfaction and fewer use of concomitant therapeutic modalities at 12 weeks suggest that combining a range of MW hyaluronic acid may be advantageous, particularly among active osteoarthritis patients.

Therefore, the HA binary mixtures of the present invention may be useful administered through intra-articular application for treatment of specific pathological conditions of the joints and in particular of traumatic and/or inflammatory joints diseases selected in the group consisting of osteoarthritis, arthrosis as well as adjuvant treatment during arthroscopy for various indications including meniscal disruption. Another application of the HA mixtures of the invention is the extra-articular applications in disorders of the soft tissues such as ligaments, said disorders being mainly strain, sprain and trauma.

The HA binary mixtures, according to their use in visco-supplementation of the synovial fluid and chondroprotection of the matrix cartilage, can be administered via intra-articular administration in pharmaceutical compositions in combination with excipients, dispersants and diluents compatible with the predictable pharmaceutical use known or even new as known to an artisan of the field. In one embodiment the mixtures of the invention can be dissolvable or dissolved in physiological isotonic aqueous solution, preferably saline, not excluding however non-electrolyte aqueous isotonic solutions. The dosages are dependent on the severity of the pathology, as well as on the state (age, body weight, general health condition) of the patient. For illustrative purposes, but not limited to, the dosages of mixtures according the invention might range between 10 and 20 mg/1ml, volume of 2 to 6 mL injection in single or in weekly repeated administration for a period ranging from 1 to 3 weeks.

## Claims

1. A binary mixture comprising two samples of hyaluronic acid in form of salt, wherein the rheological features of said binary mixture at a concentration of 10mg/ml in aqueous solution and at a temperature of 25° C are:
- a flow viscosity of a value of at least of 5 Pa/sec at a shear rate of 1/sec,
- a viscoelasticity by dynamic rheology with G' of at least of 7.5 Pa and G" of at least of 7.0 Pa at 1 Hz,
and wherein said two hyaluronic acid samples have:
a) the first sample a weight-average molecular weight lower than 1,000,000 Da,
b) the second sample a weight-average molecular weight higher than 1,500,000 Da.

2. The binary mixture according to claim 1, wherein the rheological features of said binary mixture at a concentration of 10mg/ml in aqueous solution and at a temperature of 25° C are:
- a flow viscosity of a value in a range comprised from 5 to 25 Pa/sec at a shear rate of 1/sec,
- a viscoelasticity by dynamic rheology with G' in a range comprised from 7.5 to 30 Pa and G" in a range comprised from 7.0 to 20 Pa at 1 Hz.

3. The binary mixture according to claim 2, wherein the rheological features of said binary mixture at a concentration of 10mg/ml in aqueous solution and at a temperature of 25° C are:
- a flow viscosity of a value in a range comprised from 10 to 15 Pa/sec at a shear rate of 1/sec,
- a viscoelasticity by dynamic rheology with G' in a range comprised from 10 to 15 Pa and G" in a range comprised from 7.0 to 10 Pa at 1Hz.

4. The binary mixture according to claim 1, wherein said two hyaluronic acid samples have:
a) the first sample a weight-average molecular weight in a range comprised from 300,000 up to 1,000,000 Da,
b) the second sample a weight-average molecular weight in a range comprised from 1,500,000 up to 6,000,000 Da.

5. The binary mixture according to claim 4, wherein said two hyaluronic acid samples have:
a) the first sample a weight-average molecular weight in a range comprised from 500,000 up to 900,000 Da;
b) the second sample a weight-average molecular weight in a range comprised from 1,800,000 up to 3,500,0 00 Da.

6. The binary mixture according to claim 1, wherein the hyaluronic acid in form of salt sample a) and hyaluronic acid in form of salt sample b) are in a w/w ratios in ranges comprised from 80:20 to 20:80.

7. The binary mixture according to claim 6, wherein said hyaluronic acid sample a) and said hyaluronic acid sample b) are in a w/w ratios in ranges comprised from 40:60 to 60:40.

8. The binary mixture according to claim 7, wherein said hyaluronic acid sample a) and said hyaluronic acid sample b) are in a w/w ratio of 50:50.

9. A binary mixture of two samples of hyaluronic acid in form of salt, wherein the rheological features of said binary mixture are at a concentration of 10mg/ml in aqueous solution and at a temperature of 25° C:
- a flow viscosity of a value in a range comprised from 5 to 25 Pa/sec at a shear rate of 1/sec,
- a viscoelasticity by dynamic rheology with G' in a range comprised from 7.5 to 30 Pa and G" in a range comprised from 7.0 to 20 Pa at 1 Hz
and wherein said two said hyaluronic acid samples have:
a) the first sample a weight-average molecular weight comprised in a range comprised from 300,000 up to 1,000,000 Da,
b) the second sample a weight-average molecular weight comprised in a range comprised from 1,500,000 up to 6,000,000 Da,
being said binary mixture obtainable by mixing the hyaluronic acid in form of salt sample a) and hyaluronic acid in form of salt sample b) in a w/w ratios in ranges comprised from 80:20 to 20:80.

10. The binary mixture according to claim 9, wherein said two hyaluronic acid samples have:
a) the first sample a weight-average molecular weight in a range comprised from 500,000 up to 900,000 Da;
b) the second sample a weight-average molecular weight in a range comprised from 1,800,000 up to 3,500,0 00 Da.

11. The binary mixture according to claim 9, wherein said hyaluronic acid sample a) and said hyaluronic acid sample b) are in a w/w ratios in ranges comprised from 40:60 to 60:40.

12. The binary mixture according to claim 11, wherein the said hyaluronic acid sample a) and said hyaluronic sample b) are in a w/w ratio of 50:50.

13. A pharmaceutical composition prepared with adequate diluents or excipients and binary mixtures of two samples of hyaluronic acid in form of salt according to any of the claims from 1 to 12.

14. The pharmaceutical composition according to claims 13, wherein the binary mixtures of two samples of hyaluronic acid in form of salt are in concentration in a range comprised from 10 to 20 mg/ml.

15. Use of binary mixtures of two samples of hyaluronic acid in form of salt according to any of the preceding claims from 1 to 12 for preparation of pharmaceutical compositions for treatment of inflammatory and/or traumatic joint diseases by intra-articular application.

16. Use of binary mixtures of two samples of hyaluronic acid in form of salt according to any of the preceding claims from 1 to 12 for preparation of pharmaceutical compositions for adjuvant treatment during arthroscopy by intra-articular application.

17. Use of binary mixtures of two samples of hyaluronic acid in form of salt according to claims 15 and 16, wherein said inflammatory and/or traumatic joint diseases are selected in the group consisting of osteoarthritis, arthrosis or meniscal disruption.

18. Use of binary mixtures of two samples of hyaluronic acid in form of salt according to any of the preceding claims from 1 to 12 for preparation of pharmaceutical compositions for extra-articular application in disorders of the soft tissues.

19. Use of binary mixtures of two samples of hyaluronic acid in form of salt according to claim 18, wherein said disorders are selected in the group consisting of strain, sprain and trauma.

## Patentansprüche

1. Binäre Mischung enthaltend zwei Proben von Hyaluronsäure in der Form von Salz, wobei die rheologischen Eigenschaften der binären Mischung in wässriger Lösung bei einer Konzentration von 10 mg/ml und bei einer Temperatur von 25 °C wie folgt sind:
- eine Fließviskosität mit einem Wert von wenigstens 5 Pa/Sek. bei einer Scherrate von 1/Sek.,
- eine Viskoelastizität durch dynamische Rheologie mit G' von wenigstens 7,5 Pa und mit G" von wenigstens 7,0 Pa bei 1 Hz,
und wobei die beiden Hyaluronsäureproben aufweisen:
a) die erste Probe ein gewichtsgemitteltes Molekulargewicht von weniger 1.000.000 Da und
b) die zweite Probe ein gewichtsgemitteltes Molekulargewicht von mehr als 1.500.000 Da.

2. Binäre Mischung nach Anspruch 1, wobei die rheologischen Eigenschaften der binären Mischung in wässriger Lösung bei einer Konzentration von 10 mg/ml und bei einer Temperatur von 25 °C wie folgt sind:
- eine Fließviskosität mit einem Wert in einem Bereich zwischen 5 und 25 Pa/Sek. bei einer Scherrate von 1/Sek.,
- eine Viskoelastizität durch dynamische Rheologie mit G' in einem Bereich zwischen 7,5 und 30 Pa und mit G" in einem Bereich zwischen 7,0 und 20 Pa bei 1 Hz.

3. Binäre Mischung nach Anspruch 2, wobei die rheologischen Eigenschaften der binären Mischung in wässriger Lösung bei einer Konzentration von 10 mg/ml und bei einer Temperatur von 25 °C wie folgt sind:
- eine Fließviskosität mit einem Wert in einem Bereich zwischen 10 und 15 Pa/Sek. bei einer Scherrate von 1/Sek.,
- eine Viskoelastizität durch dynamische Rheologie mit G' in einem Bereich zwischen 10 und 15 Pa und mit G" in einem Bereich zwischen 7,0 und 10 Pa bei 1 Hz.

4. Binäre Mischung nach Anspruch 1, wobei die beiden Hyaluronsäureproben aufweisen:
a) die erste Probe ein gewichtsgemitteltes Molekulargewicht in einem Bereich von 300.000 bis zu 1.000.000 Da,
b) die zweite Probe ein gewichtsgemitteltes Molekulargewicht in einem Bereich von 1.500.000 bis zu 6.000.000 Da.

5. Binäre Mischung nach Anspruch 4, wobei die beiden Hyaluronsäureproben aufweisen:
a) die erste Probe ein gewichtsgemitteltes Molekulargewicht in einem Bereich von 500.000 bis zu 900.000 Da,
b) die zweite Probe ein gewichtsgemitteltes Molekulargewicht in einem Bereich von 1.800.000 bis zu 3.500.000 Da.

6. Binäre Mischung nach Anspruch 1, wobei die Hyaluronsäure in der Form von Salz der Probe a) und die Hyaluronsäure in der Form von Salz der Probe b) in Gewichtsverhältnissen zwischen 80:20 und 20:80 vorliegen.

7. Binäre Mischung nach Anspruch 6, wobei die Hyaluronsäure der Probe a) und die Hyaluronsäure der Probe b) in Gewichtsverhältnissen zwischen 40:60 und 60:40 vorliegen.

8. Binäre Mischung nach Anspruch 7, wobei die Hyaluronsäureprobe
a) und die Hyaluronsäureprobe b) in einem Gewichtsverhältnis von 50:50 vorliegen.

9. Binäre Mischung aus zwei Proben von Hyaluronsäure in der Form von Salz, wobei die rheologischen Eigenschaften der binären Mischung in wässriger Lösung bei einer Konzentration von 10 mg/ml und bei einer Temperatur von 25 °C wie folgt sind:
- eine Fließviskosität mit einem Wert in einem Bereich zwischen 5 und 25 Pa/Sek. bei einer Scherrate von 1/Sek.,
- eine Viskoelastizität durch dynamische Rheologie mit G' in einem Bereich zwischen 7,5 und 30 Pa und mit G" in einem Bereich zwischen 7,0 und 20 Pa bei 1 Hz,
und wobei die beiden Hyaluronsäureproben aufweisen:
a) die erste Probe ein gewichtsgemitteltes Molekulargewicht in einem Bereich von 300.000 bis zu 1.000.000 Da,
b) die zweite Probe ein gewichtsgemitteltes Molekulargewicht in einem Bereich von 1.500.000 und 6.000.000 Da,
wobei die binäre Mischung durch das Vermischen der Hyaluronsäure in Form von Salz der Probe a) und der Hyaluronsäure in Form von Salz der Probe b) in Gewichtsverhältnissen zwischen 80:20 und 20:80 erhältlich ist.

10. Binäre Mischung nach Anspruch 9, wobei die beiden Hyaluronsäureproben aufweisen:
a) die erste Probe ein gewichtsgemitteltes Molekulargewicht in einem Bereich von 500.000 bis zu 900.000 Da,
b) die zweite Probe ein gewichtsgemitteltes Molekulargewicht in einem Bereich von 1.800.000 und 3.500.000 Da.

11. Binäre Mischung nach Anspruch 9, wobei die Hyaluronsäureprobe a) und die Hyaluronsäureprobe b) in Gewichtsverhältnissen zwischen 40:60 und 60:40 vorliegen.

12. Binäre Mischung nach Anspruch 11, wobei die Hyaluronsäureprobe a) und die Hyaluronsäureprobe b) in einem Gewichtsverhältnis von 50:50 vorliegen.

13. Pharmazeutische Zusammensetzung, welche aus geeigneten Verdünnungsmitteln oder Hilfsmitteln und binären Mischungen aus zwei Proben von Hyaluronsäure in Form von Salz nach einem der Ansprüche 1 bis 12 hergestellt worden sind.

14. Pharmazeutische Zusammensetzung nach Anspruch 13, wobei die binären Mischungen der beiden Proben von Hyaluronsäure in Form von Salz in einer Konzentration in einem Bereich zwischen 10 und 20 mg/ml vorliegen.

15. Verwendung von binären Mischungen aus zwei Proben von Hyaluronsäure in Form von Salz nach einem der vorhergehenden Ansprüche 1 bis 12 zur Herstellung von pharmazeutischen Zusammensetzungen für die Behandlung von entzündlichen und/oder traumatischen Gelenkserkrankungen durch intraartikuläre Anwendung.

16. Verwendung von binären Mischungen aus zwei Proben von Hyaluronsäure in Form von Salz nach einem der vorhergehenden Ansprüche 1 bis 12 zur Herstellung von pharmazeutischen Zusammensetzungen für die adjuvante Behandlung während der Arthroskopie durch intraartikuläre Anwendung.

17. Verwendung von binären Mischungen aus zwei Proben von Hyaluronsäure in Form von Salz nach Anspruch 15 oder 16, wobei die entzündlichen und/oder traumatischen Gelenkserkrankungen aus der Gruppe ausgewählt sind, welche aus Osteoarthritis, Arthrose oder Meniskusriss besteht.

18. Verwendung von binären Mischungen aus zwei Proben von Hyaluronsäure in Form von Salz nach einem der vorhergehenden Ansprüche 1 bis 12 zur Herstellung von pharmazeutischen Zusammensetzungen für die extraartikuläre Anwendung bei Krankheiten der Weichgewebe.

19. Verwendung von binären Mischungen aus zwei Proben von Hyaluronsäure in Form von Salz nach Anspruch 18, wobei die Erkrankungen aus der Gruppe ausgewählt sind, welche aus Zerrung, Verstauchung und Trauma besteht.

## Revendications

1. Mélange binaire comprenant deux échantillons d'acide hyaluronique sous forme de sel, dans lequel les particularités rhéologiques dudit mélange binaire à une concentration de 10 mg/mL dans une solution aqueuse et à une température de 25°C sont :
- une viscosité d'écoulement d'une valeur d'au moins 6 Pa/s à un taux de cisaillement de 1/s,
- une viscoélasticité par rhéologie dynamique avec G' d'au moins 7,5 Pa et G " d'au moins 7,0 Pa à 1 Hz,
et dans lequel lesdits deux échantillons d'acide hyaluronique ont :
a) pour le premier échantillon, une masse moléculaire moyenne en poids inférieure à 1 000 000 Da,
b) pour le second échantillon, une masse moléculaire moyenne en poids supérieure à 1 500 000 Da.

2. Mélange binaire selon la revendication 1, dans lequel les particularités rhéologiques dudit mélange binaire à une concentration de 10 mg/mL dans une solution aqueuse et à une température de 25 °C sont :
- une viscosité d'écoulement d'une valeur dans une plage comprise entre 5 et 25 Pa/s à un taux de cisaillement de 1/s,
- une viscoélasticité par rhéologie dynamique avec G' dans une plage comprise entre 7,5 et 30 Pa et G " dans une plage comprise entre 7,0 et 20 Pa à 1 Hz.

3. Mélange binaire selon la revendication 2, dans lequel les particularités rhéologiques dudit mélange binaire à une concentration de 10 mg/mL dans une solution aqueuse et à une température de 25 °C sont :
- une viscosité d'écoulement d'une valeur dans une plage comprise entre 10 et 15 Pa/s à un taux de cisaillement de 1/s,
- une viscoélasticité par rhéologie dynamique avec G' dans une plage comprise entre 10 et 15 Pa et G" dans une plage comprise entre 7,0 et 10 Pa à 1 Hz.

4. Mélange binaire selon la revendication 1, dans lequel lesdits deux échantillons d'acide hyaluronique ont :
a) pour le premier échantillon, une masse moléculaire moyenne en poids dans une plage comprise entre 300 000 et 1 000 000 Da,
b) pour le second échantillon, une masse moléculaire moyenne en poids dans une plage comprise entre 1 500 000 et 6 000 000 Da.

5. Mélange binaire selon la revendication 4, dans lequel lesdits deux échantillons d'acide hyaluronique ont :
a) pour le premier échantillon, une masse moléculaire moyenne en poids dans une plage comprise entre 500 000 et 900 000 Da,
b) pour le second échantillon, une masse moléculaire moyenne en poids dans une plage comprise entre 1 800 000 et 3 500 000 Da.

6. Mélange binaire selon la revendication 1, dans lequel l'échantillon a) d'acide hyaluronique sous forme de sel et l'échantillon b) d'acide hyaluronique sous forme de sel sont dans un rapport p/p dans des plages comprises entre 80 : 20 et 20 : 80.

7. Mélange binaire selon la revendication 6, dans lequel ledit échantillon a) d'acide hyaluronique et ledit échantillon b) d'acide hyaluronique sont dans un rapport p/p dans des plages comprises entre 40 : 60 et 60 : 40.

8. Mélange binaire selon la revendication 7, dans lequel ledit échantillon a) d'acide hyaluronique et ledit échantillon b) d'acide hyaluronique sont dans un rapport p/p de 50 : 50.

9. Mélange binaire de deux échantillons d'acide hyaluronique sous forme de sel, dans lequel les particularités rhéologiques dudit mélange binaire sont à une concentration de 10 mg/mL dans une solution aqueuse et à une température de 25 °C :
- une viscosité d'écoulement d'une valeur dans une plage comprise entre 5 et 25 Pa/s à un taux de cisaillement de 1/s,
- une viscoélasticité par rhéologie dynamique avec G' dans une plage comprise entre 7,5 et 30 Pa et G " dans une plage comprise entre 7,0 et 20 Pa à 1 Hz,
et dans lequel lesdits deux échantillons d'acide hyaluronique ont :
a) pour le premier échantillon, une masse moléculaire moyenne en poids comprise dans une plage comprise entre 300 000 et 1 000 000 Da,
b) pour le second échantillon, une masse moléculaire moyenne en poids comprise dans une plage comprise entre 1 500 000 et 6 000 000 Da,
ledit mélange binaire pouvant être obtenu par mélange de l'échantillon a) d'acide hyaluronique sous forme de sel et de l'échantillon b) d'acide hyaluronique sous forme de sel dans un rapport p/p dans des plages comprises entre 80 : 20 et 20 : 80.

10. Mélange binaire selon la revendication 9, dans lequel lesdits deux échantillons d'acide hyaluronique ont :
a) pour le premier échantillon, une masse moléculaire moyenne en poids dans une plage comprise entre 500 000 et 900 000 Da ;
b) pour le second échantillon, une masse moléculaire moyenne en poids dans une plage comprise entre 1 800 000 et 3 500 000 Da.

11. Mélange binaire selon la revendication 9, dans lequel ledit échantillon a) d'acide hyaluronique et ledit échantillon b) d'acide hyaluronique sont dans un rapport p/p dans des plages comprises entre 40 : 60 et 60 : 40.

12. Mélange binaire selon la revendication 11, dans lequel ledit échantillon a) d'acide hyaluronique et ledit échantillon b) d'acide hyaluronique sont dans un rapport p/p de 50 : 50.

13. Composition pharmaceutique préparée avec des diluants ou excipients adéquats et des mélanges binaires de deux échantillons d'acide hyaluronique sous forme de sel selon l'une quelconque des revendications 1 à 12.

14. Composition pharmaceutique selon la revendication 13, dans laquelle les mélanges binaires de deux échantillons d'acide hyaluronique sous forme de sel sont dans une concentration dans une plage comprise entre 10 et 20 mg/mL.

15. Utilisation de mélanges binaires de deux échantillons d'acide hyaluronique sous forme de sel selon l'une quelconque des revendications 1 à 12 précédentes pour la préparation de compositions pharmaceutiques destinées au traitement de maladies articulaires traumatiques et/ou inflammatoires par application intraarticulaire.

16. Utilisation de mélanges binaires de deux échantillons d'acide hyaluronique sous forme de sel selon l'une quelconque des revendications 1 à 12 précédentes pour la préparation de compositions pharmaceutiques destinées à un traitement d'appoint lors d'une arthroscopie par application intraarticulaire.

17. Utilisation de mélanges binaires de deux échantillons d'acide hyaluronique sous forme de sel selon les revendications 15 et 16, dans laquelle lesdites maladies articulaires traumatiques et/ou inflammatoires sont choisies dans le groupe consistant en l'ostéoarthrite, l'arthrose ou une rupture méniscale.

18. Utilisation de mélanges binaires de deux échantillons d'acide hyaluronique sous forme de sel selon l'une quelconque des revendications 1 à 12 précédentes, pour la préparation de compositions pharmaceutiques destinées à une application extraarticulaire dans les troubles des tissus mous.

19. Utilisation de mélanges binaires de deux échantillons d'acide hyaluronique sous forme de sel selon la revendication 18, dans laquelle lesdits troubles sont choisis dans le groupe consistant en la foulure, l'entorse et le traumatisme.
